# EUROPEAN PATENT SPECIFICATION

(11) **EP 2 958 551 B1**
(45) Date of publication and mention of the grant of the patent: **05.08.2020**
(21) Application number: 14754426.6
(22) Date of filing: 24.02.2014
(51) Int. Cl.: A61K 9/14, A61P 37/04

(54) **PARTICLE FORMULATIONS FOR DELIVERY OF TLR AGONISTS AND ANTIGENS**
PARTIKELFORMULIERUNGEN ZUR ABGABE VON TLR-AGONISTEN UND ANTIGENEN
FORMULATIONS PARTICULAIRES POUR L'ADMINISTRATION D'AGONISTES DU TLR ET D'ANTIGÈNES

(30) Priority: 25.02.2013 US 201361768617 P
(43) Date of publication of application: 30.12.2015
(73) Proprietor: Particle Sciences, Inc., Bethlehem, PA 18017 (US)
(72) Inventor: MITCHNICK, Mark, East Hampton, NY 11937 (US); LOXLEY, Andrew, Bethlehem, PA 18017 (US); GWOZDZ, Garry, Thomas, Nazareth, PA 18064 (US)
(74) Representative: FRKelly
(86) International application number: PCT/US2014/017935
(87) International publication number: WO 2014/130921

(56) References cited:
- WO-A1-2008/121926
- WO-A1-2012/097347
- WO-A2-2010/003009
- WO-A2-2012/054747
- US-A1- 2009 081 157
- US-A1- 2010 272 771
- US-A1- 2011 014 293
- US-B2- 8 273 361
- US-B2- 8 343 497
- MAURICIO A ARIAS ET AL: "Carnauba wax nanoparticles enhance strong systemic and mucosal cellular and humoral immune responses to HIV-gp140 antigen", VACCINE, ELSEVIER LTD, GB, vol. 29, no. 6, 24 November 2010 (2010-11-24), pages 1258-1269, XP028175837, ISSN: 0264-410X, DOI: 10.1016/J.VACCINE.2010.11.084 [retrieved on 2010-12-01]
- B.-Z. WANG ET AL: "Enhanced Influenza Virus-Like Particle Vaccines Containing the Extracellular Domain of Matrix Protein 2 and a Toll-Like Receptor Ligand", CLINICAL AND VACCINE IMMUNOLOGY, vol. 19, no. 8, 1 August 2012 (2012-08-01) , pages 1119-1125, XP055090660, ISSN: 1556-6811, DOI: 10.1128/CVI.00153-12
- David Fairhurst ET AL: "Micro-and Nano- encapsulation of Water-and Oil-soluble Actives for Cosmetic and Pharmaceutical Applications" In: "Science and Applications of Skin Delivery Systems", 1 January 2008 (2008-01-01), Allured Publishing Corporation, USA, XP055305626, * the whole document *
- ELENA STYLIANOU ET AL: "Mucosal delivery of antigen-coated nanoparticles to lungs confers protective immunity against tuberculosis infection in mice", EUROPEAN JOURNAL OF IMMUNOLOGY, vol. 44, no. 2, 4 December 2013 (2013-12-04), pages 440-449, XP055305631, DE ISSN: 0014-2980, DOI: 10.1002/eji.201343887
- CAMAUBA WAX.: 'Joint FAO/WHO Expert Committee on Food Additives.' MEETING, AND WORLD HEALTH ORGANIZATION. COMPENDIUM OF FOOD ADDITIVE SPECIFICATIONS: ADDENDUM 6. NO. 52. 1988,

## Description

This patent application claims the benefit of priority from U.S. Provisional Application Serial No. 61/768,617 filed February 25, 2013.

### Field of the Invention

The present invention relates to compositions comprising one or more Toll-like Receptor (TLR) agonists and one or more antigens adsorbed or attached to the same particles. In one embodiment, the present invention relates to a vaccine composition in which an antigen and a TLR agonist have been adsorbed or attached to the same particle. Such compositions provide for improved vaccines with enhanced immunogenicity and/or reduced reactogenicity upon administration.

### Background of the Invention

WO 2008/121926 describes pharmaceutical formulations containing particles comprised of hydrophobic organic material co-dissolved or co-dispersed with an active pharmaceutical ingredient.

U.S. Patent 5,716,637 describes nanoemulsions of particles comprising a liquid core composed of a lipid which is solid or liquid at room temperature, which is stabilized by a phospholipid envelope.

WO 2004/069227 describes a process for the preparation of a stable dispersion of solid particles, in an aqueous medium comprising by combining a pyrrole carboxamide compound, a water-miscible organic solvent and an inhibitor with an aqueous phase comprising water and a stabilizer. Bodmeier et al. (J. Microencapsulation 9(1): 89-98 (1992)) describes forming microparticles by a melt dispersion technique, in which the drug-wax melt was emulsified into a heated aqueous phase followed by cooling to form the microparticles.

Mauricio A Arias et al concerns carnauba wax nanoparticles stated to enhance strong systemic and mucosal cellular and humoral immune responses to HIV-gp140 antigen (Vaccine, vol. 29, no. 6, 2010, pages 1258 - 1269).

WO2010003009 concerns compositions that include a selected antigen, a TLR4 ligand and a TLR7/TLR8 ligand, wherein the antigen and TLR ligands are encapsulated in nanoparticles. Co-administration of both a TLR4 ligand and a TLR7/TLR8 ligand results in the synergistic induction of humor and cellular immunity as evidenced by an increase in pro-inflammatory cytokine production, an increase in the number of CD8+ T effector and T memory cells, an increase in titer of antigen-specific antibodies, an increase in antibody affinity, an increase in the proliferation of na?ve B cells and/or a significant enhancement in the persistence of antibody and T cell responses. The compositions and methods provided herein can be used to stimulate an immune response such as an immune response to a pathogen or a tumor.

### Summary of the Invention

There exists a need to provide compositions and compositions for use in methods for delivering TLR agonist and antigen combinations wherein the ratio of TLR agonist and antigen can be altered to enhance antigen immunogenicity and/or reduce TLR agonist reactogenicity.

An aspect of the present invention relates to compositions and compositions for use in methods for producing and administering compositions comprising a ratio of TLR agonists and antigens which enhance or increase tolerability of the vaccine while retaining the TLR activation activity. Compositions and compositions for use in methods of the present invention involve use of particles as a means for presenting antigens and TLR agonists in adjustable ratios which allow for optimization of immunogenicity to the antigen and/or reduction in reactogenicity to the TLR agonist. The use of particles in the compositions and compositions for use in methods of the present invention also permits non-proteinaceous antigens and existing purified vaccine antigen preparations to be combined with TLR agonists at ratios adjusted for optimization to optimize immunogenicity to the antigen and/or reduce in reactogenicity to the TLR agonist.

In one embodiment, the present invention relates to a composition comprising a TLR5 agonist, one or more antigens and a first plurality of particles to which the TLR5 agonist and the one or more antigens are attached through non-covalent interactions, said one or more antigens and said TLR5 agonist being at a ratio which increases immunogenicity to the one or more antigens and decreases reactogenicity to the TLR5 agonist, wherein the ratio of the TLR5 agonist to the antigen is less than 1:2 on a weight to weight basis.

The TLR5 agonist and one or more antigens are adsorbed or attached to the same particle.

Another aspect of the present invention relates to methods of manufacturing these particle compositions, set forth in Claims 8 and 9 of the accompanying claims.

Yet another aspect of the present invention relates to compositions for use in methods of using these particle compositions to invoke, increase and/or enhance an immune response to the antigen.

### Brief Description of the Figures

Figure 1 is a chart of the tunability of the surface charge of the particles of the invention by selection of particular emulsifiers or surfactants in the manufacture of the particles. Bars in the Figure labeled A through O stand for the following emulsifiers/surfactants: A=cationic silicone polymer; B= isostearylamidopropyldimethylamine gluconate; C=cetyltrimethylamino bromide (CTAB);D=cetylpyridinium bromide; E=dimethyldialkylammonium chloride; F=mannan; G=Brij 700; H=Brij 58; I=sodium heparin; J=gum acacia; K=disodium laurylsulfosuccinate; L=sodium lauryl sulfate; M=sodium dodecyl benzene sulfonate; N=disodium octylsulfosuccinate; and O=triethanolammonium behenate.

### Detailed Description of the Invention

The present disclosure relates to particle compositions where one or more TLR agonists and one or more antigen are attached or adsorbed to the same particle in ratios adjustable to optimize immunogenicity to the antigen and/or reduce reactogenicity to the TLR agonist. Accordingly, particle compositions of the present invention allow for TLR agonist to antigen ratios that are adjustable and differ from the 1:1 ratios required by fusion of a TLR agonist with an antigen as in the previous art. The compositions comprise particles where the ratio of the TLR5 agonist to the one or more antigens is less than 1:2 on a weight to weight basis. The disclosure mentions that the composition can comprise the TLR5 agonist and the one or more antigens located on different pluralities of particles and the ratio of TLR agonist to antigen is less than 1:2 on either a weight to weight basis or molecule to molecules basis.

By Toll-like Receptors or TLRs as used herein, it is meant to refer to the family of receptor proteins that are homologous to the *Drosophila melanogaster* Toll protein. TLRs are Type I transmembrane signaling receptor proteins characterized by an extracellular leucine-rich repeat domain and an intracellular domain homologous to an interleukin 1 receptor. TLRs include TLR1, TLR2, TLR3, TLR4, TLR5, TLR6, TLR7, TLR8, TLR9, TLR10, TLR11 and TLR12.

By "TLR agonist" for purposes of the present invention, it is meant an agent that has an affinity for and stimulates physiologic activity of a TLR normally stimulated by naturally occurring substances. For TLR1, examples of agonists include, but are not limited to, triacyl lipopeptides, glycolipids, lipopeptides and lipoproteins. For TLR2, examples of agonists include, but are not limited to, lipoteichoic acid, HSP70, and zymosan. For TLR3, examples of agonists include, but are not limited to, double-stranded RNA, poly I:C lipopolysaccharide, fibrinogen and various heat shock proteins. For TLR4, examples of agonists include, but are not limited to, fragments of heparin sulfate and hyaluronic acid, nickel and various opioids. For TLR5, examples of agonists include, but are not limited to, flagellin. For TLR6, examples of agonists include, but are not limited to, various diacyl lipopeptides and imidazoquinoline. For TLR7, examples of agonists include, but are not limited to, guanosine analogues such as loxoribine, bropirimine and single-stranded RNA. For TLR8, examples of agonists include, but are not limited to, small synthetic compounds and single-stranded RNA. For TLR9, examples of agonists include, but are not limited to, unmethylated CpG oligodeoxynucleotide DNA. For TLR10, examples of agonists include, but are not limited to, profilin. In the present disclosure, the TLR agonist has an affinity for and stimulates physiologic activity of either TLR1, TLR2, TLR3, TLR4, TLR6, TLR7, TLR8, TLR9, TLR10, TLR11 or TLR12. In the present invention, the TLR agonist has an affinity for and stimulates physiologic activity of TLR5.

Any antigen that will provoke an immune response in a human can be used in the particle compositions of the present invention in combination with a TLR agonist. By antigen, it is meant to include, but is not limited to protein, peptide, carbohydrate, glycoprotein, lipopeptide, and subunit antigens. Examples of antigens used in the compositions of the present invention include, but are not limited to, viral antigens such as influenza viral antigens (e.g. hemagglutinin (HA) protein from influenza A, B and/or C where the influenza viral hemagglutinin protein may be at least one member selected from the group consisting of H1, H2, H3, H5, H7 and H9, matrix 2 (M2) protein, neuraminidase), respiratory synctial virus (RSV) antigens (e.g. fusion protein, attachment glycoprotein), papillomaviral (e.g. human papilloma virus (HPV), such as an E6 protein, E7 protein, L1 protein and L2 protein), Herpes Simplex, rabies virus and flavivirus viral antigens (e.g. Dengue viral antigens, West Nile viral antigens), hepatitis viral antigens including antigens from HBV and HC. Antigens used in the compositions of the present invention also include, but are not limited to, bacterial antigens including those from *Streptococcus pneumonia, Haemophilus influenza, Staphylococcus aureus, Clostridium difficile* and enteric gram-negative pathogens including *Escherichia, Salmonella, Shigella, Yersinia, Klebsiella, Pseudomonas, Enterobacter, Serratia, Proteus.* Antigens used in the compositions of the present invention also include, but are not limited to fungal antigens including those from *Candida spp., Aspergillus spp., Crytococcus neoformans, Coccidiodes spp., Histoplasma capsulatum, Pneumocystis carinii, Paracoccidiodes brasiliensis, Plasmodium falciparum, Plasmodium vivax, Plasmodium ovale, and Plasmodium malariae.* Antigens for HPV vaccines would include E1, E2, E3, E4, E5, E6, E7, and the N terminus of HPVL2 alone or in combination. Antigens for RSV vaccines include, but are not limited to, RSV F or G proteins, alone or in combination. Antigens for malaria vaccines include, but are not limited to, the CSP1 protein, other pre-erythrocytic stage antigens and transmission-blocking antigens (such as, Pfs25, Pfs48 and homologues) alone or in combination. Antigens used in the present invention also include tumor antigens and/or tumor associated antigens such as, but not limited to, PSA, CEA, Mart-1, gp100, TRP-1, MAGE, NY-ESO-1, PAP, Mucin-1 and PSMA. Embodiments of the present invention also include particles with a TLR agonist and dengue antigen(s) adsorbed thereto. The Dengue disease is caused by four mosquito-borne, serologically related flaviviruses know as DEN 1 (also referred herein as Den 1), DEN 2 (Den-2), DEN 3 (Den 3) or DEN 4 (Den 4). The compositions of the invention include antigen sequences such as those sequences disclosed in WO 2009/128949 (PCT/US2009/002427), Izquierdo et al., 2008 - 814669, Mota et al., 2005, Khanam et al., 2006, Pattnaik et al., 2007, Tripathi et al., 2008; A. Zulueta, et al., Virus Research 121 (2006) 65-73. In one embodiment the antigen is T1BT, a polyoxime, constructed by chemoselective ligation, via oxime bonds, of a tetrabranched core with a peptide module containing B cell epitopes and a universal T cell epitope of the *Plasmodium falciparum* circumsporozoite protein.

Various particles can be used in the particle compositions of the present invention.

In one embodiment, lipids are used to make the particles. Examples of lipids which can be used include, but are not limited to, carnauba wax, bees wax, behenyl alcohol (docosanol), cetyl alcohol, microcrystalline triglycerides such as dynasan 118 (glyceryl tristearate) and polyethylene wax. In one embodiment, the lipid is carnauba wax including a carnauba comprising aliphatic esters, diesters of 4-hydroxycinnamic acid, ω-hydroxycarboxylic acids and fatty acid alcohols.

However, as will be understood by the skilled artisan upon reading this disclosure, particles may be comprised of non-lipids as well.

In one embodiment, the particle compositions further comprise a surfactant. Surfactants that may be used in the particle compositions include cationic, anionic and nonionic surfactants. Examples of surfactants include, but are not limited to, cetyl triammonium bromide (CTAB), N-[1-(2,3-Dioleoyloxy)]-N,N,N-trimethylammonium propane methylsulfate DOTAP, cetylpyridinium
bromide (CPB), polysorbate surfactants such as Tween 20, Tween 80 (polyoxyethylene sorbitan monoloaurate), polyethylene stearyl ether such as Brij 70, sodium stearate, sodium myristate, sodium dodecyl sulfate, Dioctyl sodium sulfosuccinate such as AOT and combinations thereof. The surfactant may present in a level from about 0.01% to about 10%, or from about 0.05% to about 5% or from about 0.1% to about 2% or from about 0.5% to about 2% or from about 1.0% to about 2.0%.

In one embodiment, the surface charge on the particle is altered to optimize attachment of the TLR agonist and/or antigen by attaching charged moieties or adjusting the type of surfactant and/or matrix used in the production of the particles.

Figure 1 demonstrates how the surface charge of the particles can be altered based on the selection of the surfactant.

Particle size is preferably less than 1µm with the particles ranging in size between about 10nm to 1000nm or between about 20nm to about 900 nm or from about 30nm to abut 800nm or from about 40nm to about 700nm or from about 50nm to about 650nm or from about 100nm to about 750nm or from about 200nm to about 750nm or from about 300nm to about 750 nm or from about 300nm to about 650nm or from about 400nm to about 750nm or from about 400nm to about 660nm or from about 500nm to about 750nm or from about 500nm to about 650nm. Particle shape may be, but is not limited to spheres, prolate and oblate spheroids, cylindrical, and irregular shapes.

Compositions of the present invention may further comprise a targeting moiety such as, but not limited to an antibody or Fabs. Such targeting moieties are also attached to the particle.

The invention further relates to methods of manufacturing these particle compositions.

In one embodiment, the method comprises a melt-emulsification-chill process in which a hot aqueous surfactant solution is added to a melted lipid, set forth in Claim 8 of the accompanying claims. The mixture is then sonicated and cooled to produce solidified lipid particles. In one embodiment, the hot aqueous surfactant solution comprises a cationic surfactant solution which is added to melted wax comprised of aliphatic esters, diesters of 4-hydroxycinnamic acid, ω-hydroxycarboxylic acids and fatty acid alcohols. A nonlimiting example of a melted wax is Yellow Carnauba wax (YC) comprised of 40% aliphatic esters, 21% diesters of 4-hydroxycinnamic acid, 13% ω-hydroxycarboxylic acids and 12% fatty acid alcohols. This process produces a solidified lipid particle having surfactant molecules oriented such that their heads are on the outer surface of the particle while the hydrophilic tails are toward the interior of the particle.

The present disclosure further relates to methods of making immunologic formulations comprising preparing a hot aqueous surfactant solution, adding the hot surfactant to a molten lipid, adding this mixture to an aqueous phase, cooling the mixture and adding TLR agonist(s) and/or antigen(s).

In the particle compositions of the present invention, the antigen(s) and/or TLR agonist(s) are absorbed or attached to the particles by non-covalent interactions such as hydrophilic or electrostatic interactions.

The particle compositions of the invention provide immunologic compositions that are more potent than the antigen alone. The particle compositions of the invention may also provide immunologic compositions in which the TLR agonist provokes a TLR mediated response, but in which the reactogenicity of the TLR agonist is reduced compared to the TLR agonist in solution when administered.

The compositions and methods of the present disclosure employ any TLR agonist in combination with an antigen. In the present invention, the TLR agonist is a TLR-5 agonist. In this embodiment, a preferred TLR-5 agonist is flagellin and in particular the type 2 flagellin of *Salmonella typhimurium,* however any of a variety of flagellins capable of binding and triggering TLR-5 may be used for this invention including engineered flagellins as described in WO 2009/128950.

Unlike the prior art methods of combining a TLR agonist with an antigen which required a one-to-one correspondence of agonist to antigen, the present invention relates to compositions that do not require a one-to-one correspondence of the agonist to antigen. In the present compositions, the TLR agonist is present in lesser amount than the antigen when both the agonist and antigen are coupled to the particle. The ratio of TLR agonist to antigen may be less than about 1:2 or, for example, may be less than about 1:3 or 1:4 or 1:5 or 1:6 or 1:7 or 1:8 or 1:9 or 1:10 or from about 1:20 or 1:30 or 1:50 or 1:100 or 1:250 or 1:500 or 1:1000 or 1:10,000 as measured by the number of molecules of TLR agonist and the number of molecules of antigen. Also the ratio of TLR agonist to antigen is less than 1:2 or may be less than about 1:3 or 1:4 or 1:5 or 1:6 or 1:7 or 1:8 or 1:9 or 1:10 or from about 1:20 or 1:30 or 1:50 or 1:100 or 1:250 or 1:500 or 1:1000 or 1:10,000 as measured by a weight to weight comparison of TLR agonist to the antigen. Conversely, the compositions of the present disclosure include those compositions where the antigen is present in an amount less than the TLR agonist. For example the ratio of antigen to the TLR agonist may be less than about 1:2 or 1:3 or 1:4 or 1:5 or 1:6 or 1:7 or 1:8 or 1:9 or 1:10 or from about 1:20 or 1:30 or 1:50 or 1:100 or 1:250 or 1:500 or 1:1000 or 1:10,000 as measured by the number of molecules of TLR agonist and the number of molecules of antigen. Also the ratio of antigen to TLR agonist may be less than about 1:2 or 1:3 or 1:4 or 1:5 or 1:6 or 1:7 or 1:8 or 1:9 or 1:10 or from about 1:20 or 1:30 or 1:50 or 1:100 or 1:250 or 1:500 or 1:1000 or 1:10,000 as measured by a weight to weight comparison of TLR agonist to the antigen.

In the present invention, the ratio of the TLR5 agonist to the antigen is less than 1:2 on a weight to weight basis.

The compositions of the present invention may comprise more than one type of antigen whether the antigen is from the same organism or a different organism. The compositions of the present invention may comprise more than one type of TLR agonist such as more than one type of flagellin from the same organism or from different organisms.

The dose of TLR agonist and antigen may be selected to optimize the immunogenic response while attempting to keep reactogenicity low. At least one dose selected from the group consisting of a 0.1µg, 0.5µg, 1µg dose, 2µg dose, 3µg dose, 4µg dose, 5µg dose, 6µg dose, 7µg dose, 8µg dose, 9µg dose, 10µg dose, 15µg dose, 20µg dose, 25µg dose and a 30µg dose may be sufficient to induce an immune response in humans. The dose of the TLR agonist and antigen may be administered to the human within a range of doses including from about 0.1µg to about 500µg, 1µg to about 100µg, 1µg to about 50µg, from about 1µg to about 30µg, from about 1µg to about 25µg, from about 1µg to about 20µg, from about 1µg to about 15µg, from about 1µg to about 10µg, from about 2µg to about 50µg, 2µg to about 30µg, from about 2µg to about 20µg, from about 2µg to about 10µg, from about 2µg to about 8µg, from about 3µg to about 50µg, 3µg to about 30µg, from about 3µg to about 20µg, from about 3µg to about 10µg, from about 3µg to about 8µg, from about 3µg to about 5µg, from about 4µg to about 50µg, 4µg to about 30µg, from about 4µg to about 20µg, from about 4µg to about 10µg, from about 4µg to about 8µg, from about 5µg to about 50µg, 5µg to about 30µg, from about 5µg to about 20µg, from about 5µg to about 10µg, from about 5µg to about 9µg, and from about 5µg to about 8µg.

With respect to compositions comprising a TLR agonist and antigen, the dosage refers to the amount of total protein present in the vaccine given to the human wherein some of the protein quantity relates to the antigen and some of the protein quantity relates to the TLR agonist.

The immunogenic compositions for use according to the present disclosure may be delivered as a standard 0.01 - 2.0 ml injectable dose and contain from about 0.1µg to about 50µg of antigen. In a preferred disclosure of the immunogenic compositions for use according to the present invention is a 0.1 ml injectable dose and contains about 1µg of antigen, 1.4µg of carnauba wax and 0.14µg of surfactant. The vaccine volume may be between 0.05 and 1.0 ml or between about 0.05 and 0.5ml or about 0.10 to about 0.25 ml. A vaccine dose according to the present invention may be provided in a smaller volume than conventional dosing. Low volume doses according to the present invention are suitably below 0.5ml, typically below 0.3ml and usually not less than 0.01 ml.

Thus, the present invention provides compositions and compositions for use in methods for optimizing the antigenicity (i.e. increasing the antigenicity) and/or the reactogenicity (i.e. decreasing the reactogenicity) of TLR agonist/antigen combinations as composed on single particles, including antigen/TLR agonist combinations that have been poorly immunogenic and/or highly reactogenic. The immunogenic particle compositions of the present disclosure comprising one or more TLR agonists and one or more antigens may be more immunogenic than the combination of TLR agonist and antigen as a fusion protein. The immunogenic particle compositions of the present invention comprising a TLR5 agonist and one or more antigens may be more immunogenic than the combination of TLR agonist and antigen as a fusion protein. For example, the immunogenicity of the antigen as measured by the antibody response to the antigen may be greater for the particle compositions of the present invention than the antigen alone in solution or as a TLR agonist-antigen fusion by greater than 10% or greater than about 20% or greater than about 30% or greater than about 40% or greater than about 50% or greater than about 60% or greater than about 70% or greater than about 80% or greater than about 90% or greater than about 100% or greater than about 200% or greater than about 500% or greater than about 1000%. The immunogenic particle compositions comprising one or more TLR agonists and one or more antigens may be less reactogenic than the combination of TLR agonist and antigen as a fusion protein. For example, the reactogenicity to the TLR agonist as measured by the antibody response to the TLR agonist may be less for the particle compositions of the present invention than the antigen alone in solution or as a TLR agonist-antigen fusion by less than about 10% or less than about 20% or less than about 30% or less than about 40% or less than about 50% or less than about 60% or less than about 70% or less than about 80% or less than about 90% or less than about 100% or less than about 200% or less than about 500% or less than about 1000%. While the TLR agonist is less reactogenic in the compositions of the present invention, the particle compositions of the present invention still maintain the ability to trigger a TLR agonist response.

Accordingly, the compositions and compositions for use in methods of the present invention are useful in production of vaccine formulations to invoke an immune response in a human. Further, the compositions of the present invention are useful in increasing antibody response to an antigen, even a poorly antigenic antigen, in an animal in order to generate therapeutic and or diagnostic antibodies to be extracted and purified.

Ranges may be expressed herein as from about one particular value, and/or to about another particular value. When such a range is expressed, another aspect includes from the one particular value and/or to the other particular value. Similarly, when values are expressed as approximations, by use of the antecedent about, it will be understood that the particular value forms another aspect. It will be further understood that the endpoints of each of the ranges are significant both in relation to the other endpoint, and independently of the other endpoint. Where ranges are given herein, the endpoints are included. Furthermore, it is to be understood that unless otherwise indicated or otherwise evident from the context and understanding of one of ordinary skill in the art, values that are expressed as ranges can assume any specific value or sub range within the stated ranges in different embodiments of the invention, to the tenth of the unit of the lower limit of the range, unless the context clearly dictates otherwise.

Within this disclosure, any indication that a feature is optional is intended provide adequate support (e.g., under 35 U.S.C. 112 or Art. 83 and 84 of EPC) for claims that include closed or exclusive or negative language with reference to the optional feature. Exclusive language specifically excludes the particular recited feature from including any additional subject matter. For example, if it is indicated that A can be drug X, such language is intended to provide support for a claim that explicitly specifies that A consists of X alone, or that A does not include any other drugs besides X. "Negative" language explicitly excludes the optional feature itself from the scope of the claims. For example, if it is indicated that element A can include X, such language is intended to provide support for a claim that explicitly specifies that A does not include X. Non-limiting examples of exclusive or negative terms include "only," "solely," "consisting of," "consisting essentially of," "alone," "without", "in the absence of (e.g., other items of the same type, structure and/or function)" "excluding," "not including", "not", "cannot," or any combination and/or variation of such language.

Similarly, referents such as "a," "an," "said," or "the," are intended to support both single and/or plural occurrences unless the context indicates otherwise. For example "a dog" is intended to include support for one dog, no more than one dog, at least one dog, a plurality of dogs, etc. Non-limiting examples of qualifying terms that indicate singularity include "a single", "one," "alone", "only one," "not more than one", etc. Non-limiting examples of qualifying terms that indicate (potential or actual) plurality include "at least one," "one or more," "more than one," "two or more," "a multiplicity," "a plurality," "any combination of," "any permutation of," "any one or more of," etc. Claims or descriptions that include "or" between one or more members of a group are considered satisfied if one, more than one, or all of the group members are present in, employed in, or otherwise relevant to a given product or process unless indicated to the contrary or otherwise evident from the context.

The citation of any publication is for its disclosure prior to the filing date and should not be construed as an admission that the present invention is not entitled to antedate such publication by virtue of prior invention.

While this invention has been particularly shown and described with references to example embodiments thereof, it will be understood by those skilled in the art that the various changes in form and details may be made therein without departing from the scope of the invention encompassed by the appended claims.

### EXAMPLES

### Example 1: Manufacture of Particle Formulations

### Selection of Antigen and TLR agonist

Antigen A is selected from antigens for any of the infectious disease listed in Table 1 (also see en.wikipedia with the extension .org/wiki/List_of_infectious_diseases of the world wide web).

**Table 1: List of infectious diseases in Humans**

| **DISEASE** | **SOURCE OF DISEASE** |
|---|---|
| *Acinetobacter* infections | *Acinetobacter baumannii* |
| Actinomycosis | *Actinomyces israelii, Actinomyces gerencseriae* and *Propionibacterium propionicus* |
| African sleeping sickness (African trypanosomiasis) | *Trypanosoma brucei* |
| AIDS (Acquired immunodeficiency syndrome) | HIV (Human immunodeficiency virus) |
| Amebiasis | *Entamoeba histolytica* |
| Anaplasmosis | *Anaplasma* genus |
| Anthrax | *Bacillus anthracis* |
| *Arcanobacterium haemolyticum* infection | *Arcanobacterium haemolyticum* |
| Argentine hemorrhagic fever | Junin virus |
| Ascariasis | *Ascaris lumbricoides* |
| Aspergillosis | *Aspergillus* genus |
| Astrovirus infection | Astroviridae family |
| Babesiosis | *Babesia* genus |
| *Bacillus cereus* infection | *Bacillus cereus* |
| Bacterial pneumonia | multiple bacteria |
| Bacterial vaginosis (BV) | multiple bacteria |
| *Bacteroides* infection | *Bacteroides* genus |
| Balantidiasis | *Balantidium coli* |
| *Baylisascaris* infection | *Baylisascaris* genus |
| BK virus infection | BK virus |
| Black piedra | *Piedraia hortae* |
| *Blastocystis hominis* infection | *Blastocystis hominis* |
| Blastomycosis | *Blastomyces dermatitidis* |
| Bolivian hemorrhagic fever | Machupo virus |
| *Borrelia* infection | *Borrelia* genus |
| Botulism (and Infant botulism) | *Clostridium botulinum;* Note: Botulism is not an infection by *Clostridium botulinum* but caused by the intake of botulinum toxin. |
| Brazilian hemorrhagic fever | Sabia |
| Brucellosis | *Brucella* genus |
| *Burkholderia* infection | usually *Burkholderia cepacia* and other *Burkholderia* species |
| Buruli ulcer | Mycobacterium ulcerans |
| Calicivirus infection (Norovirus and Sapovirus) | Caliciviridae family |
| Campylobacteriosis | *Campylobacter* genus |
| Candidiasis (Moniliasis; Thrush) | usually *Candida albicans* and other *Candida* species |
| Cat-scratch disease | *Bartonella henselae* |
| Cellulitis | usually Group A *Streptococcus* and *Staphylococcus* |
| Chagas Disease (American trypanosomiasis) | *Trypanosoma cruzi* |
| Chancroid | *Haemophilus ducreyi* |
| Chickenpox | Varicella zoster virus (VZV) |
| Chlamydia | *Chlamydia trachomatis* |
| *Chlamydophila pneumoniae* infection | *Chlamydophila pneumoniae* |
| Cholera | *Vibrio cholerae* |
| Chromoblastomycosis | usually *Fonsecaea pedrosoi* |
| Clonorchiasis | *Clonorchis sinensis* |
| *Clostridium difficile* infecti*on* | *Clostridium difficile* |
| Coccidioidomycosis | *Coccidioides immitis* and *Coccidioides posadasii* |
| Colorado tick fever (CTF) | Colorado tick fever virus (CTFV) |
| Common cold (Acute viral rhinopharyngitis; Acute coryza) | usually rhinoviruses and coronaviruses. |
| Creutzfeldt-Jakob disease (CJD) | CJD prion |
| Crimean-Congo hemorrhagic fever (CCHF) | Crimean-Congo hemorrhagic fever virus |
| Cryptococcosis | *Cryptococcus neoformans* |
| Cryptosporidiosis | *Cryptosporidium* genus |
| Cutaneous larva migrans (CLM) | usually *Ancylostoma braziliense*; multiple other parasites |
| Cyclosporiasis | *Cyclospora cayetanensis* |
| Cysticercosis | *Taenia solium* |
| Cytomegalovirus infection | Cytomegalovirus |
| Dengue fever | Dengue viruses (DEN-1, DEN-2, DEN-3 and DEN-4) - Flaviviruses |
| Dientamoebiasis | *Dientamoeba fragilis* |
| Diphtheria | *Corynebacterium diphtheriae* |
| Diphyllobothriasis | *Diphyllobothrium* |
| Dracunculiasis | *Dracunculus medinensis* |
| Ebola hemorrhagic fever | Ebolavirus (EBOV) |
| Echinococcosis | *Echinococcus* genus |
| Ehrlichiosis | *Ehrlichia* genus |
| Enterobiasis (Pinworm infection) | *Enterobius vermicularis* |
| *Enterococcus* infection | *Enterococcus* genus |
| Enterovirus infection | Enterovirus genus |
| Epidemic typhus | *Rickettsia prowazekii* |
| Erythema infectiosum (Fifth disease) | Parvovirus B19 |
| Exanthem subitum (Sixth disease) | Human herpesvirus 6 (HHV-6) and Human herpesvirus 7 (HHV-7) |
| Fasciolopsiasis | *Fasciolopsis buski* |
| Fasciolosis | *Fasciola hepatica* and *Fasciola gigantica* |
| Fatal familial insomnia (FFI) | FFI prion |
| Filariasis | Filarioidea superfamily |
| Food poisoning by *Clostridium perfringens* | *Clostridium perfringens* |
| Free-living amebic infection | multiple |
| *Fusobacterium* infection | *Fusobacterium* genus |
| Gas gangrene (Clostridial myonecrosis) | usually *Clostridium perfringens*; other *Clostridium* species |
| Geotrichosis | *Geotrichum candidum* |
| Gerstmann-Straussler-Scheinker syndrome (GSS) | GSS prion |
| Giardiasis | *Giardia intestinalis* |
| Glanders | *Burkholderia mallei* |
| Gnathostomiasis | *Gnathostoma spinigerum* and *Gnathostoma hispidum* |
| Gonorrhea | Neisseria gonorrhoeae |
| Granuloma inguinale (Donovanosis) | *Klebsiella granulomatis* |
| Group A streptococcal infection | *Streptococcus pyogenes* |
| Group B streptococcal infection | *Streptococcus agalactiae* |
| *Haemophilus influenzae* infection | *Haemophilus influenzae* |
| Hand, foot and mouth disease (HFMD) | Enteroviruses, mainly Coxsackie A virus and Enterovirus 71 (EV71) |
| Hantavirus Pulmonary Syndrome (HPS) | Sin Nombre virus |
| *Helicobacter pylori* infection | *Helicobacter pylori* |
| Hemolytic-uremic syndrome (HUS) | *Escherichia coli* 0157:H7, 0111 and 0104:H4 |
| Hemorrhagic fever with renal syndrome (HFRS) | Bunyaviridae family |
| Hepatitis A | Hepatitis A Virus |
| Hepatitis B | Hepatitis B Virus |
| Hepatitis C | Hepatitis C Virus |
| Hepatitis D | Hepatitis D Virus |
| Hepatitis E | Hepatitis E Virus |
| Herpes simplex | Herpes simplex virus 1 and 2 (HSV-1 and HSV-2) |
| Histoplasmosis | *Histoplasma capsulatum* |
| Hookworm infection | *Ancylostoma duodenale* and *Necator americanus^{.}* |
| Human bocavirus infection | Human bocavirus (HBoV) |
| Human ewingii ehrlichiosis | *Ehrlichia ewingii* |
| Human granulocytic anaplasmosis (HGA) | *Anaplasma phagocytophHum* |
| Human metapneumovirus infection | Human metapneumovirus (hMPV) |
| Human monocytic ehrlichiosis | *Ehrlichia chaffeensis* |
| Human papillomavirus (HPV) infection | Human papillomavirus (HPV) |
| Human parainfluenza virus infection | Human parainfluenza viruses (HPIV) |
| Hymenolepiasis | *Hymenolepis nana* and Hymenolepis diminuta |
| Epstein-Barr Virus Infectious Mononucleosis (Mono) | Epstein-Barr Virus (EBV) |
| Influenza (flu) | Orthomyxoviridae family |
| Isosporiasis | *Isospora belli* |
| Kawasaki disease | unknown; evidence supports that it is infectious |
| Keratitis | multiple |
| *Kingella kingae* infection | *Kingella kingae* |
| Kuru | Kuru prion |
| Lassa fever | Lassa virus |
| Legionellosis (Legionnaires' disease) | *Legionella pneumophila* |
| Legionellosis (Pontiac fever) | *Legionella pneumophila* |
| Leishmaniasis | *Leishmania* genus |
| Leprosy | *Mycobacterium leprae* and *Mycobacterium lepromatosis* |
| Leptospirosis | *Leptospira* genus |
| Listeriosis | *Listeria monocytogenes* |
| Lyme disease (Lyme borreliosis) | usually *Borrelia burgdorferi* and other *Borrelia* species |
| Lymphatic filariasis (Elephantiasis) | *Wuchereria bancrofti* and *Brugia malayi* |
| Lymphocytic choriomeningitis | Lymphocytic choriomeningitis virus (LCMV) |
| Malaria | *Plasmodium* genus |
| Marburg hemorrhagic fever (MHF) | Marburg virus |
| Measles | Measles virus |
| Melioidosis (Whitmore's disease) | *Burkholderia pseudomallei* |
| Meningitis | multiple |
| Meningococcal disease | *Neisseria meningitidis* |
| Metagonimiasis | usually *Metagonimus yokagawai* |
| Microsporidiosis | Microsporidia phylum |
| Molluscum contagiosum (MC) | Molluscum contagiosum virus (MCV) |
| Mumps | Mumps virus |
| Murine typhus (Endemic typhus) | *Rickettsia typhi* |
| Mycoplasma pneumonia | *Mycoplasma pneumoniae* |
| Mycetoma | numerous species of bacteria (Actinomycetoma) and fungi (Eumycetoma) |
| Myiasis | parasitic dipterous fly larvae |
| Neonatal conjunctivitis (Ophthalmia neonatorum) | most commonly *Chlamydia trachomatis* and *Neisseria gonorrhoeae* |
| (New) Variant Creutzfeldt-Jakob disease (vCJD, nvCJD) | vCJD prion |
| Nocardiosis | usually *Nocardia asteroides* and other *Nocardia* species |
| Onchocerciasis (River blindness) | *Onchocerca volvulus* |
| Paracoccidioidomycosis (South American blastomycosis) | *Paracoccidioides brasiliensis* |
| Paragonimiasis | usually *Paragonimus westermani* and other *Paragonimus* species |
| Pasteurellosis | *Pasteurella* genus |
| Pediculosis capitis (Head lice) | *Pediculus humanus capitis* |
| Pediculosis corporis (Body lice) | *Pediculus humanus corporis* |
| Pediculosis pubis (Pubic lice, Crab lice) | *Phthirus pubis* |
| Pelvic inflammatory disease (PID) | multiple |
| Pertussis (Whooping cough) | *Bordetella pertussis* |
| Plague | *Yersinia pestis* |
| Pneumococcal infection | *Streptococcus pneumoniae* |
| Pneumocystis pneumonia (PCP) | *Pneumocystis jirovecii* |
| Pneumonia | multiple |
| Poliomyelitis | Poliovirus |
| *Prevotella* infection | *Prevotella* genus |
| Primary amoebic meningoencephalitis (PAM) | usually *Naegleria fowleri* |
| Progressive multifocal leukoencephalopathy | JC virus |
| Psittacosis | *Chlamydophila psittaci* |
| Q fever | *Coxiella burnetii* |
| Rabies | Rabies virus |
| Rat-bite fever | *Streptobacillus moniliformis* and *Spirillum minus* |
| Respiratory syncytial virus infection | Respiratory syncytial virus (RSV) |
| Rhinosporidiosis | *Rhinosporidium seeberi* |
| Rhinovirus infection | Rhinovirus |
| Rickettsial infection | *Rickettsia* genus |
| Rickettsialpox | *Rickettsia akari* |
| Rift Valley fever (RVF) | Rift Valley fever virus |
| Rocky mountain spotted fever (RMSF) | *Rickettsia rickettsii* |
| Rotavirus infection | Rotavirus |
| Rubella | Rubella virus |
| Salmonellosis | *Salmonella* genus |
| SARS (Severe Acute Respiratory Syndrome) | SARS coronavirus |
| Scabies | *Sarcoptes scabiei* |
| Schistosomiasis | *Schistosoma* genus |
| Sepsis | multiple |
| Shigellosis (Bacillary dysentery) | *Shigella* genus |
| Shingles (Herpes zoster) | Varicella zoster virus (VZV) |
| Smallpox (Variola) | Variola major or Variola minor |
| Sporotrichosis | *Sporothrix schenckii* |
| Staphylococcal food poisoning | *Staphylococcus* genus |
| Staphylococcal infection | *Staphylococcus* genus |
| Strongyloidiasis | *Strongyloides stercoralis* |
| Syphilis | *Treponema pallidum* |
| Taeniasis | *Taenia* genus |
| Tetanus (Lockjaw) | *Clostridium tetani* |
| Tinea barbae (Barber's itch) | usually *Trichophyton* genus |
| Tinea capitis (Ringworm of the Scalp) | usually *Trichophyton tonsurans* |
| Tinea corporis (Ringworm of the Body) | usually *Trichophyton* genus |
| Tinea cruris (Jock itch) | usually *Epidermophyton floccosum, Trichophyton rubrum,* and *Trichophyton mentagrophytes* |
| Tinea manuum (Ringworm of the Hand) | *Trichophyton rubrum* |
| Tinea nigra | usually *Hortaea werneckii* |
| Tinea pedis (Athlete's foot) | usually *Trichophyton* genus |
| Tinea unguium (Onychomycosis) | usually *Trichophyton* genus |
| Tinea versicolor (Pityriasis versicolor) | *Malassezia* genus |
| Toxocariasis (Ocular Larva Migrans (OLM)) | *Toxocara canis* or *Toxocara cati* |
| Toxocariasis (Visceral Larva Migrans (VLM)) | *Toxocara canis* or *Toxocara cati* |
| Toxoplasmosis | *Toxoplasma gondii* |
| Trichinellosis | *Trichinella spiralis* |
| Trichomoniasis | *Trichomonas vaginalis* |
| Trichuriasis (Whipworm infection) | *Trichuris trichiura.* |
| Tuberculosis | usually *Mycobacterium tuberculosis* |
| Tularemia | *Francisella tularensis* |
| *Ureaplasma urealyticum* infection | *!!HYPERLINK* |
| Venezuelan equine encephalitis | Venezuelan equine encephalitis virus |
| Venezuelan hemorrhagic fever | Guanarito virus |
| Viral pneumonia | multiple viruses |
| West Nile Fever | West Nile virus |
| White piedra (Tinea blanca) | *Trichosporon beigelii* |
| *Yersinia pseudotuberculosis* infection | *Yersinia pseudotuberculosis* |
| Yersiniosis | *Yersinia enterocolitica* |
| Yellow fever | Yellow fever virus |
| Zygomycosis | Mucorales order (Mucormycosis) and Entomophthorales order (Entomophthoramycosis) |

TLR agonist B is selected from those listed in Table 2 (also see Zuany-Amorim et al. Nature Reviews Drug Discovery 2002 1: 797-807).

**Table 2: Toll-like Receptors**

| TLR-family member | Exogenous Ligands |
|---|---|
| TLR-1(can associate with TLR-2) | Mycobacterial lipoprotein, triacylated lipopeptides |
| TLR-2 (can associate with TLR-1 or TLR-6) | LPS, yeast-particle zymosan, peptidoglycan (bacteria), lipoproteins (bacteria and mycoplasmas), GPI anchor from *Trypanosoma cruzi* |
| TLR-3 | Poly(I:C)(viral dsRNA) |
| TLR-4 | LPS, respiratory syncytial virus |
| TLR-5 | Flagellin |
| TLR-6 (can associate with TLR-2) | Mycoplasma lipoproteins, lipoteichoic acid, peptidoglycan (bacteria) |
| TLR-7 | Unknown; synthetic compounds such as resiquimod or imiquimod, activate the receptor |
| TLR-8 | Unknown; synthetic compounds such as resiquimod or imiquimod, activate the receptor |
| TLR-9 | CpG DNA |
| TLR-10 | Unknown |

A composition comprising a TLR5 agonist, one or more antigens and a first plurality of particles to which the TLR5 agonist and the one or more antigens are attached through non-covalent interactions forms the present invention.

### Manufacture of Antigen A and TLR Agonist B particles

Stock cationic particles are prepared by a melt-emulsification-chill process as follows. Yellow Carnauba wax (YC), 10 grams, comprised of 40% aliphatic esters, 21% diesters of 4-hydroxycinnamic acid, 13% ω-hydroxycarboxylic acids and 12% fatty acid alcohols is melted in a 250 mL glass beaker at 90°C. In a second 250 mL glass beaker, 90 mL of 1% cationic surfactant solution (either cetyl triammonium bromide (CTAB) or cetylpyridinium
bromide (CPB)) is heated to 90°C. The hot aqueous surfactant solution is added to the molten YC wax with tip sonication for 3 minutes to form an emulsion. The emulsion is then rapidly cooled using an ice-bath with an overhead stirrer. A 1% w/w dispersion is prepared by diluting this emulsion stock with water.

The selected TLR Agonist B solution is prepared by dilution with water to achieve either 1 mg/mL or 10 mg/mL working solution.

The Antigen A solution is prepared in a 10% DMSO/water mixture and used immediately to avoid antigen degradation.

The required volumes of the TLR Agonist B solution and the Antigen A solution in order to achieve the desired final concentration are pipetted into a sterile 2 mL plastic vial, VIAL 1, and mixed by inversion. The required volume of 1% w/w cationic particle dispersion and the required volume of water consistent with the desired final concentration is pipetted into a separate sterile 2 mL plastic vial, VIAL 2, and mixed by inversion. Each final formulation is prepared by transferring by Eppendorf pipette, with drop-wise addition, all the solutions in VIAL 1 to VIAL 2. The final mixture in VIAL 2 is then inverted three times to yield the final Antigen A and TLR Agonist B Particle formulation ready for use.

## Claims

1. A composition comprising a TLR5 agonist, one or more antigens and a first plurality of particles to which the TLR5 agonist and the one or more antigens are attached through non-covalent interactions, said one or more antigens and said TLR5 agonist being at a ratio which increases immunogenicity to the one or more antigens and decreases reactogenicity to the TLR5 agonist, wherein the ratio of the TLR5 agonist to the antigen is less than 1:2 on a weight to weight basis.

2. The composition of claim 1 wherein the particles are comprised of a lipid.

3. The composition of claim 2, wherein the lipid is selected from the group consisting of Dynasan118 / PEG35-castor oil blend, natural Carnauba wax, synthetic carnauba wax, Bees wax, a self-emulsifying wax, polyethylene wax, behenyl alcohol and oleic acid/compritol.

4. The composition of claim 2, wherein the lipid is carnauba wax, optionally wherein the carnauba wax comprises aliphatic esters, diesters of 4-hydroxycinnamic acid ω-hydroxycarboxylic acids and fatty acid alcohols.

5. The composition of any of claims 1 through 4 further comprising a surfactant; optionally
wherein the surfactant is selected from the group consisting of cetyl triammonium bromide (CTAB), N-[1-(2,3-Dioleoyloxy)]-N,N,N-trimethylammonium propane methylsulfate DOTAP, cetylpyridinium
bromide (CPB), Tween 20, Tween 80 (polyoxyethylene sorbitan monoloaurate), Brij 70, sodium stearate, sodium myristate, sodium dodecyl sulfate, dioctyl sodium sulfosuccinate and combinations thereof.

6. The composition of any of claims 1 through 5 further comprising a targeting moiety.

7. The composition of any of claims 1 through 6 wherein the ratio of the TLR5 agonist to the antigen is less than 1:20 on a weight to weight basis.

8. A method of making the composition of claim 5 when dependent on any of claims 2 to 4 when dependent on claim 1 comprising:
(a) preparing a hot aqueous surfactant solution;
(b) adding the hot surfactant to a molten lipid to form a hot lipid/surfactant mixture;
(c) adding the hot lipid/surfactant mixture to an aqueous phase to form a second mixture;
(d) sonicating the second mixture;
(e) cooling the second mixture; and
(f) adding a TLR5 agonist and one or more antigens to form a composition comprising particles to which the TLR5 agonist and one or more antigens is attached through non-covalent interactions.

9. The method of claim 8 wherein the surfactant is cetyl triammonium bromide; or
wherein the surfactant is cetylpyridinium bromide.

10. The composition of any of claims 1 through 7 wherein the antigen is at least 10% more immunogenic than the antigen alone in solution; or
wherein the antigen is at least 10% more immunogenic than the antigen fused to the TLR agonist; or
wherein the TLR agonist is at least 10% less reactogenic than the TLR agonist alone in solution; or
wherein the TLR agonist is at least 10% less reactogenic than the antigen fused to the TLR agonist.

11. The composition of any of claims 1 through 7 for use in a method for inducing an immune response in a human, said method comprising administering the composition to the human.

12. The composition of any of claims 1 through 7 for use in a method for increasing antibody response to a poorly antigenic antigen in an animal, said method comprising administering the composition to the animal.

## Patentansprüche

1. Zusammensetzung, die Folgendes umfasst: einen TLR5-Agonisten, ein oder mehrere Antigene und eine erste Vielzahl von Partikeln, an die der TLR5-Agonist und das eine oder die mehreren Antigene durch nichtkovalente Wechselwirkungen gebunden sind, wobei das eine oder die mehreren Antigene und der TLR5-Agonist in einem Verhältnis vorliegen, das die Immunogenität gegenüber dem einen oder den mehreren Antigenen erhöht und die Reaktogenität gegenüber dem TLR5-Agonisten erniedrigt, wobei das Verhältnis des TLR5-Agonisten zu dem Antigen kleiner als 1 : 2, bezogen auf das Gewicht, ist.

2. Zusammensetzung nach Anspruch 1, wobei die Partikel aus einem Lipid bestehen.

3. Zusammensetzung nach Anspruch 2, wobei das Lipid aus der Gruppe ausgewählt ist, die aus Folgenden besteht: Dynasan118/PEG35-Rizinusöl-Gemisch, natürlichem Carnaubawachs, synthetischem Carnaubawachs, Bienenwachs, selbstemulgierendem Wachs, Polyethylenwachs, Behenylalkohol und Ölsäure/Compritol.

4. Zusammensetzung nach Anspruch 2, wobei das Lipid Carnaubawachs ist, optional wobei das Carnaubawachs aliphatische Ester, Diester von 4-Hydroxyzimtsäure, ω-Hydroxycarbonsäuren und Fettsäurealkoholen umfasst.

5. Zusammensetzung nach einem der Ansprüche 1 bis 4, die weiter ein Tensid umfasst, optional
wobei das Tensid aus der Gruppe ausgewählt ist, die aus Folgenden besteht: Cetyltriammoniumbromid (CTAB), N-[1-(2,3-Dioleoyloxy)]-N,N,N-trimethylammoniumpropanmethylsulfat DOTAP, Cetylpyridiniumbromid (CPB), Tween 20, Tween 80 (Polyoxyethylensorbitanmonolaurat), Brij 70, Natriumstearat, Natriummyristat, Natriumdodecylsulfat, Dioctylnatriumsulfosuccinat und Kombinationen davon.

6. Zusammensetzung nach einem der Ansprüche 1 bis 5, die weiter eine zielgerichtete Einheit umfasst.

7. Zusammensetzung nach einem der Ansprüche 1 bis 6, wobei das Verhältnis des TLR5-Agonisten zu dem Antigen kleiner als 1 : 20, bezogen auf das Gewicht, ist.

8. Verfahren zur Herstellung der Zusammensetzung nach Anspruch 5, bei Abhängigkeit von einem der Ansprüche 2 bis 4, bei Abhängigkeit von Anspruch 1, das Folgendes umfasst:
(a) Herstellen einer heißen wässrigen Tensidlösung;
(b) Zugeben des heißen Tensids zu einem geschmolzenen Lipid, um eine heiße Lipid/Tensid-Mischung zu bilden;
(c) Zugeben der heißen Lipid/Tensid-Mischung zu einer wässrigen Phase, um eine zweite Mischung zu bilden;
(d) Ultraschallbehandlung der zweiten Mischung;
(e) Abkühlen der zweiten Mischung; und
(f) Zugeben eines TLR5-Agonisten und eines oder mehrerer Antigene, um eine Zusammensetzung zu bilden, die Partikel umfasst, an die der TLR5-Agonist und das eine oder die mehreren Antigene durch nichtkovalente Wechselwirkungen gebunden sind.

9. Verfahren nach Anspruch 8, wobei das Tensid Cetyltriammoniumbromid ist; oder wobei das Tensid Cetylpyridiniumbromid ist.

10. Zusammensetzung nach einem der Ansprüche 1 bis 7, wobei das Antigen mindestens 10 % immunogener ist als das Antigen allein in Lösung; oder
wobei das Antigen mindestens 10 % immunogener ist als das Antigen, das mit dem TLR-Agonisten fusioniert ist; oder
wobei der TLR-Agonist mindestens 10 % weniger reaktogen ist als der TLR-Agonist allein in Lösung; oder
wobei der TLR-Agonist mindestens 10 % weniger reaktogen ist als das Antigen, das mit dem TLR-Agonisten fusioniert ist.

11. Zusammensetzung nach einem der Ansprüche 1 bis 7 für die Verwendung in einem Verfahren zur Induzierung einer Immunreaktion bei einem Menschen, wobei das Verfahren das Verabreichen der Zusammensetzung an den Menschen umfasst.

12. Zusammensetzung nach einem der Ansprüche 1 bis 7 für die Verwendung in einem Verfahren zur Erhöhung der Antikörperreaktion auf ein schwach antigenes Antigen bei einem Tier, wobei das Verfahren das Verabreichen der Zusammensetzung an das Tier umfasst.

## Revendications

1. Composition comprenant un agoniste de TLR5, un ou plusieurs antigènes et une première pluralité de particules auxquelles l'agoniste de TLR5 et les un ou plusieurs antigènes sont fixés via des interactions non covalentes, lesdits un ou plusieurs antigènes et ledit agoniste de TLR5 se trouvant selon un rapport qui augmente l'immunogénicité vis-à-vis des un ou plusieurs antigènes et diminue la réactogénicité vis-à-vis de l'agoniste de TLR5, où le rapport de l'agoniste de TLR5 à l'antigène est inférieur à 1:2 selon une base pondérale.

2. Composition selon la revendication 1, dans laquelle les particules sont constituées d'un lipide.

3. Composition selon la revendication 2, dans laquelle le lipide est choisi dans le groupe constitué par un mélange de Dynasan 118/huile de ricin de PEG 35, la cire de carnauba naturelle, la cire de carnauba synthétique, la cire d'abeille, une cire auto-émulsifiante, une cire de polyéthylène, l'alcool béhénylique, et l'acide oléique/Compritol.

4. Composition selon la revendication 2, dans laquelle le lipide est constitué de cire de carnauba, éventuellement où la cire de carnauba comprend des esters aliphatiques, des diesters d'acide 4-hydroxycinnamique, des acides ω-hydroxycarboxyliques et des alcools d'acide gras.

5. Composition selon l'une quelconque des revendications 1 à 4, comprenant en outre un agent tensioactif ; éventuellement où l'agent tensioactif est choisi dans le groupe constitué par le bromure de cétyltriammonium (CTAB), le méthylsulfate de N-[l-(2,3-dioléyloxy)propyl]-N,N,N-triméthylammonium (DOTAP), le bromure de cétylpyridinium (CPB), le Tween 20, le Tween 80 (monolaurate de polyoxyéthylène sorbitane), le Brij 70, le stéarate de sodium, le myristate de sodium, le dodécylsulfate de sodium, le dioctylsulfosuccinate de sodium, et des combinaisons de ceux-ci.

6. Composition selon l'une quelconque des revendications 1 à 5, comprenant en outre un motif de ciblage.

7. Composition selon l'une quelconque des revendications 1 à 6, dans laquelle le rapport de l'agoniste de TLR5 à l'antigène est inférieur à 1:20 selon une base pondérale.

8. Méthode de préparation de la composition selon la revendication 5 lorsque dépendante de l'une quelconque des revendications 2 à 4 lorsque dépendantes de la revendication 1, comprenant :
(a) la préparation d'une solution d'agent tensioactif aqueuse chaude ;
(b) l'addition de l'agent tensioactif chaud à un lipide fondu afin de former un mélange lipide/agent tensioactif chaud ;
(c) l'addition du mélange lipide/agent tensioactif chaud à une phase aqueuse afin de former un deuxième mélange ;
(d) le traitement aux ultrasons du deuxième mélange ;
(e) le refroidissement du deuxième mélange ; et
(f) l'addition d'un agoniste de TLR5 et d'un ou plusieurs antigènes afin de former une composition comprenant des particules auxquelles l'agoniste de TLR5 et un ou plusieurs antigènes sont fixés via des interactions non covalentes.

9. Méthode selon la revendication 8, dans laquelle l'agent tensioactif est le bromure de cétyltriammonium ; ou
où l'agent tensioactif est le bromure de cétylpyridinium.

10. Composition selon l'une quelconque des revendications 1 à 7, dans laquelle l'antigène est au moins 10% plus immunogène que l'antigène seul en solution ; ou
où l'antigène est au moins 10% plus immunogène que l'antigène fusionné avec l'agoniste de TLR ; ou
où l'agoniste de TLR est au moins 10% moins réactogène que l'agoniste de TLR seul en solution ; ou
où l'agoniste de TLR est au moins 10% moins réactogène que l'antigène fusionné avec l'agoniste de TLR.

11. Composition selon l'une quelconque des revendications 1 à 7, pour une utilisation dans une méthode destinée à l'induction d'une réponse immunitaire chez un être humain, ladite méthode comprenant l'administration de la composition à l'être humain.

12. Composition selon l'une quelconque des revendications 1 à 7, pour une utilisation dans une méthode destinée à l'augmentation d'une réponse d'anticorps vis-à-vis d'un antigène peu antigénique chez un animal, ladite méthode comprenant l'administration de la composition à l'animal.
